# EUROPEAN PATENT SPECIFICATION

(11) **EP 0 929 517 B1**
(45) Date of publication and mention of the grant of the patent: **12.06.2002**
(21) Application number: 97909339.0
(22) Date of filing: 30.09.1997
(51) Int. Cl.: C07C 259/06, A61K 31/16

(54) **HYDROXAMIC ACID DERIVATIVES**
HYDROXAMSÄURE-DERIVATE
DERIVES D'ACIDE HYDROXAMIQUE

(30) Priority: 02.10.1996 GB 9620572; 02.04.1997 GB 9706667
(43) Date of publication of application: 21.07.1999
(73) Proprietor: Novartis AG, 4056 Basel (CH)
(72) Inventor: KOTTIRSCH, Georg, D-79111 Freiburg (DE); NEUMANN, Ulf, CH-4310 Rheinfelden (CH)
(74) Representative: Becker, Konrad
(86) International application number: EP9705376
(87) International publication number: WO9814424

(56) References cited:
- WO-A-94/10990
- WO-A-95/19956
- WO-A-95/19961
- WO-A-96/06074
- WO-A-96/16931

## Description

This invention relates to novel hydroxamic acid compounds which are useful as pharmaceuticals, e.g., in inhibiting matrix metalloproteinases such as collagenase, and in inhibiting TNF production, particularly for treatment of diseases or conditions mediated by over-production of or over-responsiveness to TNFα.

Tumor necrosis factor (TNF) is a cytokine which is produced initially as a membrane-bound 28 kD precursor. It is then cleaved by an enzyme (TNF convertase) and released as a soluble, active 17 kD form. Soluble TNF exists in at least two forms, TNFα and TNFβ, of which TNFα appears to be the more significant clinically. TNFα is believed to mediate inflammation and other conditions associated with septic shock or acute infections. Long term overstimulation by TNFα is believed to play a role in autoimmune and chronic inflammatory conditions, such as arthritis, multiple sclerosis, and the like.

It has been shown that certain matrix metalloproteinase inhibitors of the hydroxamic acid class, in particular 3-imino-4-oxo-heptane-1,7-dioic acid (7-N-hydroxy) diamides (which are optionally further 1-N-, 2-, 5-, and 6- substituted) are capable of mediating TNFα production, possibly by inhibiting TNF convertase. Known representatives of this class of compounds are summarized and described, e.g., in WO 94/10990.

It has now surprisingly been discovered that a new class of hydroxamic acid derivatives ("Novel Compounds") are potent TNFα suppressors and have advantageous pharmaceutical properties, in particular, oral bioavailability.

The Novel Compounds are 3-imino-4-oxo-6-(oxymethyl)-heptane-1,7-dioic acid (7-N-hydroxy) diamides. Suitably, the 6-oxymethyl substituent is of formula II below, e.g., hydroxymethyl or mono- or polyalkoxymethyl. The Novel Compounds may have further substitutions at the 1-N-, 2-, and 5- positions as known in the art, e.g., as described in WO 94/10990, or as further described herein. For example, the Novel Compounds may be 1-N substituted with methyl, pyridyl, or a substituent of formula X-Y- or X'-Y- as described below, e.g., 3-imino-4-oxo-6-(oxymethyl)-heptane-1,7-dioic acid (1-N-morpholinocarbonylalkyl, 7-N-hydroxy) diamide, and may be in free or pharmaceutically acceptable salt form.

A particularly preferred class of Novel Compounds are 3-imino-4-oxo-5-aryl-6-(oxymethyl)-heptane-1,7-dioic acid (7-N-hydroxy) diamides. The 5-aryl substituent may be as further described herein, e.g. wherein the 5-aryl substituent is phenyl optionally substituted, conveniently at the 4-position, e.g. by hydroxy-, C₁₋₆ alkyl-, C₁₋₆ alkoxy-, amino-, halo- or cyano-. Such 5-aryl substituted Novel Compounds may be in free or pharmaceutically acceptable salt form.

Preferably, the Novel Compounds are of Formula I 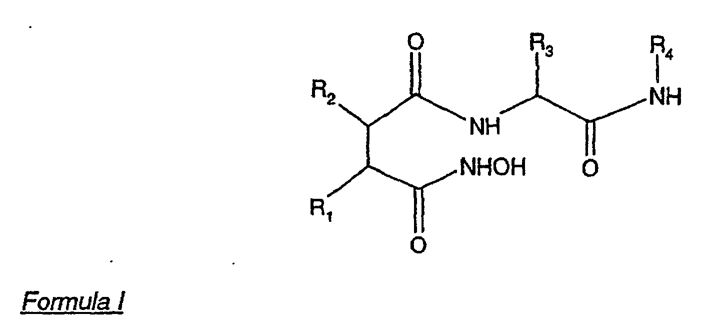 wherein
- **R**_{**1**} is: a substituent of Formula II:

A-(O-(CR₅H)ₙ)ₘ-O-CH₂- *Formula II*

wherein
**n** is 1, 2, 3 or 4, preferably 2;
**m** is 0, 1, 2 or 3;
each **R**_{**5**} is
independently H, C₁₋₁₀ (optionally hydroxy-, C₁₋₆ alkoxy-, amino-, C₁₋₆ alkylamino-, thiol-, C₁₋₆ alkylmercapto- or protected hydroxy, amino or thiol substituted) alkyl, C₂₋₆ alkenyl, C₆₋₁₄(optionally hydroxy-, C₁₋₆ alkoxy-, amino-, C₁₋₆ alkylamino-, halo- or cyano- substituted) aryl, or C₆₋₁₄ (aryl) C₁₋₆alkyl; preferably H, phenyl, benzyl or C₁₋₅ alkyl;
A is hydrogen, C₁₋₁₀ alkyl, C₆₋₁₄ aryl, C₆₋₁₄ aryl(C₁₋₆ alkyl), (C₆₋₁₄ aryl)carbonyl, or (C₁₋₁₀ alkyl)carbonyl; preferably hydrogen, C₁₋₆ alkyl (e.g., methyl or cyclohexyl), phenyl or benzyl;
- **R**_{**2**} is: C₂₋₁₂ alkyl, C₃₋₁₂ alkenyl, C₃₋₇(optionally hydroxy-, C₁₋₆ alkoxy-, amino-, or C₁₋₆ alkylamino- substituted) cycloalkyl, C₅₋₁₄ aryl, or C₅₋₁₄ aryl(C₁₋₆ alkyl), wherein aryl groups are optionally substituted by hydroxy-, C₁₋₆ alkyl-, C₁₋₆ alkoxy-, amino-, halo-or cyano-; preferably phenyl, 4-methylphenyl, 4-methoxyphenyl, cyclohexyl or isobutyl;
- **R**_{**3**} is: C₁₋₁₀ (optionally hydroxy- or C₁₋₆alkoxy- amino-, C₁₋₆ alkylamino-, thiol-, C₁₋₆ alkylmercapto- or protected hydroxy-, amino- or thiol- substituted) alkyl (e.g., *t*-butyl, or cyclohexylmethyl), C₆₋₁₄ (optionally hydroxy-, C₆₋₁₄aryloxy-, or C₁₋₆alkoxy-, amino-, C₁₋₆ alkylamino-, halo-, or cyano- substituted) aryl (e.g., benzyl, *p*-methoxybenzyl, *p*-benzyloxybenzyl), or indolylmethyl (e.g., 2-indolylmethyl); preferably benzyl or *t*-butyl;
- **R**_{**4**} is: methyl, pyridyl, or a substituent of formula X-Y- wherein X is morpholino, pyridyl or aryl (preferably morpholino), and Y is C₁₋₁₂alkylene in which up to four of the methylene (-CH₂-) units are optionally replaced with -CO-, -NH-, -SO₂- or -O-; for example methyl, 2-pyridyl, morpholinocarbonylmethyl, 5-(morpholino)pentyl, or 5-(morpholinocarbonyl)pentyl.
- "alkyl": includes linear, cyclic, or branched alkyl; and
- "aryl": refers to an monovalent aromatic radical containing one or two aromatic rings, e.g., phenyl, benzyl, or tolyl, and includes heteroaryl containing one or more hetero atoms, e.g. N, O or S.

Halo or halogen as used herein refers to F, Cl; Br or I unless otherwise indicated.

Conveniently R₁ is a substituent of formula II'

A-(O-(CH₂)ₙ)ₘ-O-CH₂- *Formula II'*

wherein A, n and m are as defined above.

In an alternative particular embodiment R₁ is a substituent of formula II"

A-O-(CHR₅-(CH₂)ₙ)_{m'}-O-CH₂- *Formula II"*

wherein A, n and R₅ are as defined above and m' is 0, 1 or 2.

When R₄ of formula I is a substituent of formula X-Y-, it is preferably a substituent of formula X'-Y- wherein X' is morpholino and Y is as defined above.

In particular embodiments the invention provides Novel Compounds of formula I in which independently:
**n** of Formula II is 3 or 4; or
**R**_{**5**} of Formula II is not H; or
**R**_{**2**} is C₇₋₁₂ alkyl, C₃₋₁₂ alkenyl, C₃₋₇(optionally hydroxy-, C₁₋₆ alkoxy-, amino-, or C₁₋₆ alkylamino- substituted) cycloalkyl, C₅₋₁₄ aryl, or C₅₋₁₄ aryl(C₁₋₆ alkyl), wherein aryl groups are optionally substituted by hydroxy-, C₁₋₆ alkyl-, C₁₋₆ alkoxy-, amino-, halo- or cyano-; preferably phenyl, 4-methylphenyl, 4-methoxyphenyl or cyclohexyl; or
**R**_{**3**} is C₁₋₁₀(amino-, C₁₋₆ alkylamino-, thiol-, C₁₋₆ alkylmercapto- or protected hydroxy-, amino- or thiol- substituted)alkyl, C₆₋₁₄(amino-, C₁₋₆ alkylamino-, halo-, or cyano-substituted)aryl; or
any aryl group thereof is heteroaryl containing one or more hetero atoms, e.g. N, O or S.

In further particular embodiments the invention provides Novel Compounds of formula I' 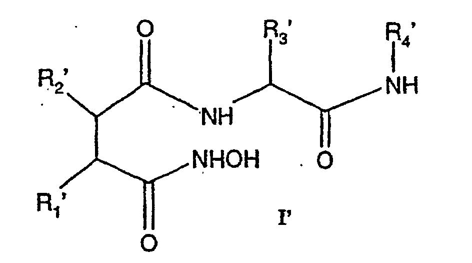 in which
- **R**_{**1**}**'** is: a substituent of Formula II''':

A'-(O-(CH₂)_{n'})_{m'}-O-CH₂- *Formula II'''*

such that
**n'** is an integer one or two, preferably two;
**m'** is an integer zero, one, two, or three;
**A'** is hydrogen, C₆₋₁₄ aryl, C₁₋₁₀ alkyl, (C₆₋₁₄ aryl)carbonyl, or (C₁₋₁₀ alkyl)carbonyl, (preferably C₁₋₆ alkyl, e.g., methyl or cyclohexyl);
- **R**_{**2**}**'** is: C₂₋₆ alkyl, preferably isobutyl;
- **R**_{**3**}**'** is: C₁₋₁₀ (optionally hydroxy- or C₁₋₆alkoxy-substituted) alkyl (e.g., *t*-butyl, or cyclohexylmethyl), C₆₋₁₄ (optionally hydroxy-, C₆₋₁₄aryloxy-, or C₁₋₆alkoxy-substituted) aryl (e.g., benzyl, *p*-methoxybenzyl, *p*-benzyloxybenzyl), or indolylmethyl (e.g., 2-indolylmethyl); preferably benzyl or *t*-butyl;
- **R**_{**4**}**'** is: methyl, pyridyl, or a substituent of formula X-Y- wherein X is morpholino, pyridyl or aryl (preferably morpholino), and Y is C₁₋₁₂alkylene in which up to four of the methylene (-CH₂-) units are optionally replaced with -CO-, -NH-, -SO₂- or -O-; for example methyl, 2-pyridyl, morpholinocarbonylmethyl, 5-(morpholino)pentyl, or 5-(morpholinocarbonyl)pentyl;

Preferred Novel Compounds in which **R**_{**2**} is aryl are compounds in which **R**_{**1**} is of formula II' as defined above and **R**_{**2**} is phenyl, 4-methylphenyl or 4-methoxyphenyl.

An especially preferred group of compounds of Formula I are compounds wherein:
(i) **R**_{**1**} is of formula II' or II" (preferably formula II') and A of formula II is hydrogen, C₁₋₆ alkyl, e.g., methyl or cyclohexyl (e.g., so that R₁ of formula I is for example hydroxymethyl, cyclohexyloxyethoxymethyl, methoxyethoxyethoxymethyl, or hydroxyethyloxymethyl) or (C₆₋₁₄ aryl)carbonyl, e.g. benzoyl (e.g. so that R₁ of formula I is for example benzoyloxymethyl, benzoyloxyethoxyethyl or benzoyloxyethoxyethoxymethyl);
(ii) R₂ of formula I is cyclohexyl, phenyl, 4-methylphenyl, 4-methoxyphenyl or isobutyl;
(iii) R₃ of formula I is benzyl or *t*-butyl; and
(iv) R₄ of formula I is methyl or morpholinocarbonyl(C₁₋₆)alkyl.

The Novel Compounds may exist in free or salt forms, and salt forms are intended to be encompassed by the scope of the invention. For example, certain of the Novel Compounds may exist as physiologically acceptable acid or base addition salts, e.g. as chlorhydrates, oxalates or fumarates.

The configuration of the Novel Compounds is preferably that of Formula Ia: 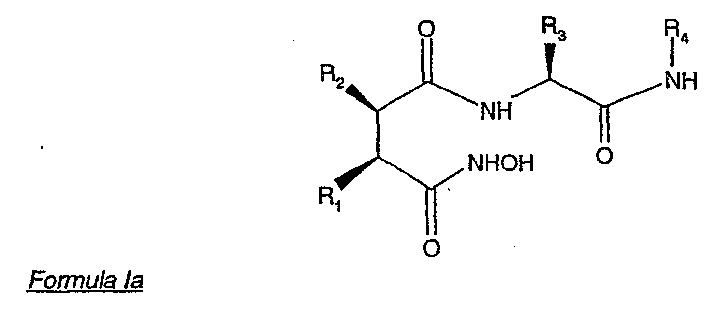 or of Formula Ib: 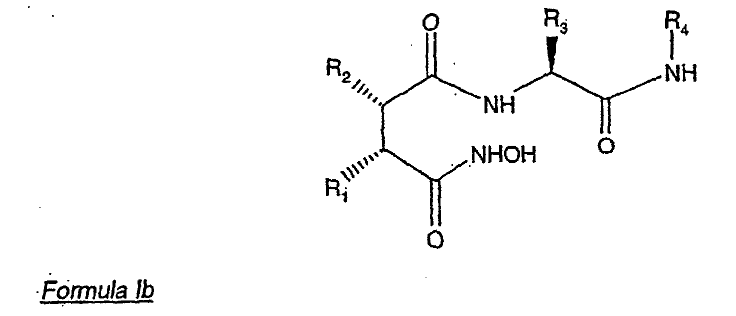 most preferably that of Formula Ia.

Thus the invention includes Novel Compounds when in the form of mixtures of enantiomers, e.g. as racemic mixtures, though preferably when in pure or substantially pure enantiomeric form, e.g. in a form in which the Novel Compound content comprises at least 90%, preferably at least 95%, and especially at least 98%, of a single isomer (i.e. comprises less than 10%, preferably less than 5%, and especially less than 2%, of other Novel Compound isomers.

In further aspects, the invention provides novel processes for the preparation of a compound of formula I as defined above comprising reacting a compound of Formula III: 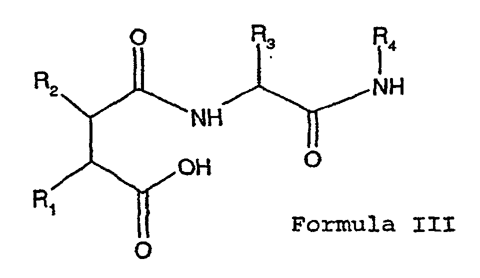 wherein R₁, R₂, R₃ and R₄ are as defined above, with hydroxylamine (optionally in salt or O-substituted form, e.g., hydroxylamine hydrochloride), recovering the product of formula I, and optionally deprotecting the product or separating the desired diastereoisomer if required,

A compound of Formula III as defined above may be prepared by oxidizing the olefin bond of a compound of formula IV: 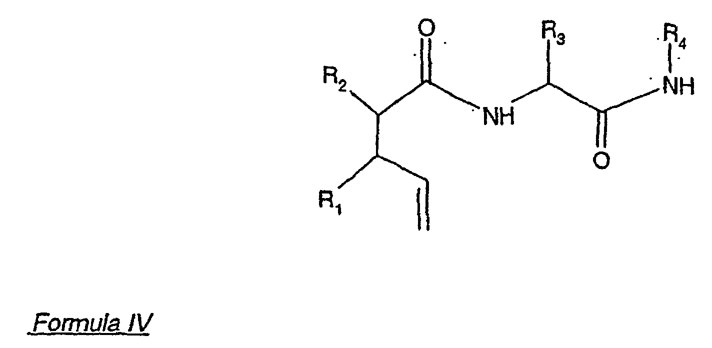 wherein R₁, R₂, R₃ and R₄ are as defined above, e.g., using an oxidation catalyst such as ruthenium(III)chloride hydrate, to obtain the acid of Formula III, and optionally separating the desired diastereoisomer if required.

A compound of formula IV may be prepared by reacting a carboxylic acid of formula V

CH₂=CH-CH(R₁)-CH(R₂)-COOH *Formula V*

wherein R₁ and R₂ are as defined above, with an amino acid amide of formula VI

NH₂-CH(R₃)-CO-NH(R₄) *Formula VI*

wherein R₃ and R₄ are as defined above, to yield an amide corresponding to formula IV, and optionally separating the desired diastereoisomer if required.

A compound of formula V may be prepared by reacting an alcohol of formula II^{IV}

A"-(O-(CR₅H)ₙ)ₘ-OH *Formula II*^{*IV*}

wherein A" is as defined above for A of formula II, except that when A is H, A" is an O-protecting group (for example a group capable of forming a readily cleavable ether, e.g., benzyl), and wherein R₅; n and m are as defined for Formula II above, with a dihalogenated alkene (trans), e.g., 1,4-dibromobut-2-ene, to obtain the disubstituted R₁, halo-alkene, e.g., R₁-CH=CH-CH₂-W (trans), where W is halogen, e.g., bromine, which is then reacted with a carboxylic acid corresponding to R₂, i.e, R₂-CH₂COOH, to yield the ester, which is then rearranged, e.g., in the presence of an organic base such as lithium diisopropyl amide, to give the compound of formula V.

Optionally, protecting and deprotecting steps may be included in the above described processes as necessary to preserve the integrity of the intermediates and the final product.

The invention further includes *per se* the novel intermediates of formulae III and IV as defined above.

As discussed in the test examples below, the Novel Compounds are potent inhibitors of TNFα release, are orally active, and are not cytotoxic at their effective doses. The Novel Compounds also inhibit collagenase and stromelysin at concentrations of from 0.3 to 10 nM. The Novel Compounds tested further show oral activity in vivo at dosages of less than 10 mg /kg in LPS induced TNFα release in the rat, and appear to be well tolerated at such dosages. Accordingly, the Novel Compounds have pharmaceutical utility as follows:

The Novel Compounds are useful for the prophylaxis and treatment of diseases or pathological conditions mediated by TNF, especially TNFα, e.g., inflammatory conditions, autoimmune diseases, severe infections, and organ or tissue transplant rejection, e.g. for the treatment of recipients of heart, lung, combined heart-lung, liver, kidney, pancreatic, skin or corneal transplants and for the prevention of graft-versus-host disease, such as following bone marrow transplants.

The Novel Compounds are particularly useful for the treatment, prevention, or amelioration of autoimmune disease and of inflammatory conditions, in particular inflammatory conditions with an aetiology including an autoimmune component such as arthritis (for example rheumatoid arthritis, arthritis chronica progrediente and arthritis deformans) and rheumatic diseases. Specific auto-immune diseases for which the Novel Compounds may be employed include autoimmune haematological disorders (including e.g. hemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, psoriasis, Steven-Johnson syndrome, idiopathic sprue, autoimmune inflammatory bowel disease (including e.g. ulcerative colitis and Crohn's disease), endocrine ophthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

The Novel Compounds are also useful for the treatment, prevention, or amelioration of asthma, bronchitis, pneumoconiosis, pulmonary emphysema, and other obstructive or inflammatory diseases of the airways.

The Novel Compounds are useful for treating undesirable acute and hyperacute inflammatory reactions which are mediated by TNF, especially by TNFα, e.g., acute infections, for example septic shock (e.g., endotoxic shock and adult respiratory distress syndrome), meningitis, pneumonia; and severe burns; and for the treatment of cachexia or wasting syndrome associated with morbid TNF release, consequent to infection, cancer, or organ dysfunction, especially AIDS -related cachexia, e.g., associated with or consequential to HIV infection.

In addition to inhibiting the release of TNF, especially TNFα through the suppression of TNF convertase, the Novel Compounds are also inhibitors of matrix metalloproteinases, e.g., collagenase, stromelysin and gelatinases, and hence useful for the indications known for collagenase inhibitors or other matrix metalloproteinase inhibitors, e.g., treatment of various pathological conditions of the skin, bones, and connective tissues, e.g., rheumatoid arthritis, psoriasis, psoriatic arthritis, osteoporosis, osteoarthritis, periodontitis, gingivitis, and corneal ulceration; for the treatment of cardiovascular disease, e.g., atherosclerosis, and coronary angioplasty; for the prevention of tumor cell metastasis and invasion and in inducing fibrosis of tumors, e.g., in the treatment of cancer; and for the prevention of neurodegenerative disorders, e.g., Alzheimer's disease.

For the above indications the appropriate dosage will, of course, vary depending, for example, on the particular Novel Compound employed, the subject to be treated, the mode of administration and the nature and severity of the condition being treated. However, in general, satisfactory results in animals are obtained at daily dosages of from about 1 to about 10mg/kg/day p.o.. In larger mammals, for example humans, an indicated daily dosage is in the range of from about 50 to about 750mg of Novel Compound administered orally once or, more suitably, in divided dosages two to four times/day.

The Novel Compounds may be administered by any conventional route, e.g. orally, for example in the form of solutions for drinking, tablets or capsules or parenterally, for example in the form of injectable solutions or suspensions. Normally for systemic administration oral dosage forms are preferred, although for some indications the Novel Compounds may also be administered topically or dermally, e.g. in the form of a dermal cream or gel or like preparation or, for the purposes of application to the eye, in the form of an ocular cream, gel or eye-drop preparation; or may be administered by inhalation, e.g., for treating asthma. Suitable unit dosage forms for oral administration comprise e.g. from 25 to 250mg Novel Compound per unit dosage.

In accordance with the foregoing the present invention also provides in a further series of embodiments:
A. A Novel Compound for use as a pharmaceutical, e.g. for use as an immunosuppressant or antiinflammatory agent or for use in the prevention, amelioration or treatment of any disease or condition as described above, e.g., an autoimmune or inflammatory disease or condition.
B. A pharmaceutical composition comprising a Novel Compound in association with a pharmaceutically acceptable diluent or carrier, e.g., for use as an immunosuppressant or anti-inflammatory agent or for use in the prevention, amelioration or treatment of any disease or condition as described above, e.g., an autoimmune or inflammatory disease or condition.
C. Use of a Novel Compound in the manufacture of a medicament for use as an immunosuppressant or anti-inflammatory agent or for use in the prevention, amelioration or treatment of any disease or condition as described above, e.g., an autoimmune of inflammatory disease or condition.

### EXAMPLES

### Example 1 : Preparation of R-2-iso-butyl-S-3-(2,5,8-trioxanonyl)-succinic acid-{1-[S-phenyl alanine-1-methylamide]-4-[N-hydroxyl]}-diamide (the compound of formula I wherein R₁ is 2,5,8,-Trioxanonyl, R₂ is isobutyl, R₃ is benzyl, and R₄ is methyl)

a. A solution of trans-1,4-dibromo-2-butene (CAS Reg. 821-06-7) (50.00 g) , diethylene glycol monomethyl ether (CAS Reg. 111-77-3) (30.89 g), tetra-butylammonium hydrogen sulfate (7.94 g) (CAS Reg. 32503-27-8) and 50% aqueous sodium hydroxide solution (113.70 ml) in methylene chloride (200 ml) is stirred at room temperature (r.t.) for 16 h. The reaction mixture is diluted with water and ether, the organic phase is separated and the product olefin is purified by chromatography.
b. A solution of the trans-olefin product of step a (27.11 g) and DBU
   (1,8-diazabicyclo[5.4.0]undec-7-ene, CAS Reg. 6674-22-2)(17.6 ml) in methylene chloride (200 ml) is treated with isocaproic acid (12.44 g). After one hour, anhydrous sodium carbonate (18 g) is added. The mixture is kept overnight. The organic phase is separated and the product ester is purified by chromatography.
c. A solution of LDA (lithium diisopropyl amide) in tetrahydrofuran (400 ml) is prepared at -70°C from diisopropylamine (22.65 ml) and butyl lithium in hexane (1.6 N) (99.89 ml). A solution of the product of step b (43.90 g) in tetrahydrofuran (100 ml) is added at the same temperature. After 30 minutes, chlorotrimethylsilane (20.22 ml) is added. The temperature is raised first to room temperature then to reflux overnight. The mixture is diluted with ether. The non-acidic products are removed from the organic phase to give 35.12 g of crude acid which is then chromatographed to give 30.70 g pure carboxylic acid product.
d. A solution of the product of step c (10.50 g), (L) L-phenyl alanine-1-methyl amide (8.60 g) (e.g., preferably prepared by reacting commercially available N-carbobenzoxy (L) phenyl alanine with methylamine under standard conditions to obtain the methyl amide and hydrogenating in the presence of palladium to deprotect the amino group), and 4-dimethylaminopyridine (4.89 g) in methylene chloride (120 ml) is treated with EDCI (1-(3-dimethylaminopropyl)-3-ethylcarbodiimide hydrochloride, CAS Reg. 25952-53-8) (7.68 g) and triethyl amine (7.61 ml) and kept overnight. Ether is added, and the organic phase is dried and evaporated. The crude product, a mixture of two isomers, is chromatographed on silica gel to separate the isomers by their relative polarity.
e. The vigorously stirred solution of the less polar product of step d (5.30 g) in carbon tetrachloride (150 ml), acetonitrile (150 ml) and water (20 ml) is treated with ruthenium(III)chloride hydrate (0.49 g) and sodium perhydrate (15.16 g). Ether is added after two hours and the pH is adjusted to 4. The organic phase is separated, dried and evaporated. The residue is chromatographed on silica gel to give the pure acid.
f. A solution of the product of step e (5 g), hydroxybenzytriazole (2.00 g) and EDCI (2.51 g) in DMF (N,N-dimethyl formamide)(20 ml) is kept at room temperature for 2.5 hours. Hydroxylamine hydrochloride (1.90 g) and N-methylmorpholine (4.61 ml) are then added, and the mixture is left overnight. The solvent is evaporated under high vacuum at 50°C. The residue is purified by HPLC on RP18-silica gel to give the pure hydroxamic acid as white crystaline powder.
   Melting point: 195 - 197 °C; Optical rotation: [α]_{D}²⁰ = -8.5° c=0.175 in MeOH.

### Examples 2-17:

The compounds corresponding to examples 2 - 17 of table I are prepared in analogy to example 1. The product of step c in example 1 is reacted with the the appropriate amino acid amide derivatives as decribed in step d of example 1. Following the procedures of step e and f of example 1 give the pure hydroxamic acids.

### Examples 18-32:

Gyclbhexylglycol is used in lieu of diethylene glycol monomethyl ether for reaction with trans-1,4-dibromo-2-butene as described in step a of example 1. Following the procedures as described in step b - f of example 1 gives the pure hydroxamic acids of example 18-32 of table I.

### Examples 33 and 34

Monobenzylglycol or (2-Benzyl) glycol monbenzyl ether is used in lieu of glycol monomethyl etherfor reaction with trans 1,4-dibromo-2-butene as described in step a) of Example 1. Following the same procedures as described in steps b) to f) of Example 1 gives the hydroxamic acids of formula I having a benzyl protected hydroxy group at R1. Hydrogenation in the presence of a catalytic amount of paladium or barium sulphate removes the benzyl group, thus yielding after HPLC purification on RP18 silica gel the corresponding pure compounds of formula I (see Table I).

### Examples 35-59:

Benzyl-alcohol is used in lieu of diethylene glycol monomethyl ether for reaction with trans-1,4-dibromo-2-butene as described in step a of example 1. Following the same procedures as described in step b - f of example 1 with appropriate adjustment of starting reagent, quantities etc. gives the hydroxamic acids of formula I having benzyloxymethyl at R₁. Hydrogenation in the presence of catalytic amounts of palladium on barium sulfate removes the benzyl, thus yielding, after purification on HPLC on RP18 silica gel, the pure products of example 35 - 59 of Table I.

**TABLE I**

| Ex¹ | R₁ | R₂ | R₃ | R₄ |
|---|---|---|---|---|
| 1 | CH₃(OCH₂CH₂)₂OCH₂ | isobutyl | benzyl | methyl |
| 2 | CH₃(OCH₂CH₂)₂OCH₂ | isobutyl | benzyl | 2-(morpholino)ethyl |
| 3 | CH₃(OCH₂CH₂)₂OCH₂ | isobutyl | benzyl | 5-(p-tosylamino) pentyl |
| 4 | CH₃(OCH₂CH₂)₂OCH₂ | isobutyl | benzyl | 2-(morpholino carbonyl)ethyl |
| 5 | CH₃(OCH₂CH₂)₂OCH₂ | isobutyl | benzyl | 2-(p-tosylamino)ethyl |
| 6 | CH₃(OCH₂CH₂)₂OCH₂ | isobutyl | benzyl | 1-S-(methyl-carbamoyl)ethyl |
| 7 | CH₃(OCH₂CH₂)₂OCH₂ | isobutyl | tert.butyl | methyl |
| 8 | CH₃(OCH₂CH₂)₂OCH₂ | isobutyl | tert.butyl | morpholinocarbonylmethyl |
| 9 | CH₃(OCH₂CH₂)₂OCH₂ | isobutyl | tert.butyl | 2-(morpholino carbonyl)ethyl |
| 10 | CH₃(OCH₂CH₂)₂OCH₂ | isobutyl | tert.butyl | 5-(morpholino carbonyl)pentyl |
| 11 | CH₃(OCH₂CH₂)₂OCH₂ | isobutyl | tert.butyl | 2-pyridyl |
| 12 | CH₃(OCH₂CH₂)₂OCH₂ | isobutyl | p-methoxybenzyl | methyl |
| 13 | CH₃(OCH₂CH₂)₂OCH₂ | isobutyl | 1-R-tert. butyloxy-ethyl | methyl |
| 14 | CH₃(OCH₂CH₂)₂OCH₂ | isobutyl | 1-R-benzyloxyethyl | methyl |
| 15 | CH₃(OCH₂CH₂)₂OCH₂ | isobutyl | p-benzyloxybenzyl | methyl |
| 16 | CH₃(OCH₂CH₂)₂OCH₂ | isobutyl | methyl-3-indolyl | methyl |
| 17 | CH₃(OCH₂CH₂)₂OCH₂ | isobutyl | 1-R-hydroxyethyl | methyl |
| 18 | c-hexyl-OCH₂CH₂OCH₂ | isobutyl | benzyl | methyl |
| 19 | c-hexyl-OCH₂CH₂OCH₂ | isobutyl | benzyl | 2-pyridyl |
| 20 | c-hexyl-OCH₂CH₂OCH₂ | isobutyl | benzyl | 3,6-dioxa-8-oxo-9-imino-11-morpholino-undecyl |
| 21 | c-hexyl-OCH₂CH₂OCH₂ | isobutyl | benzyl | 5-(morpholino)pentyl |
| 22 | c-hexyl-OCH₂CH₂OCH₂ | isobutyl | benzyl | 4-(morpholino)butyl |
| 23 | c-hexyl-OCH₂CH₂OCH₂ | isobutyl | benzyl | 3,5-dioxa-8-oxo-8-morpholino-octyl |
| 24 | c-hexyl-OCH₂CH₂OCH₂ | isobutyl | tert.butyl | methyl |
| 25 | c-hexyl-OCH₂CH₂OCH₂ | isobutyl | tert.butyl | 6-imino-8-phenyl-octyl |
| 26 | c-hexyl-OCH₂CH₂OCH₂ | isobutyl | tert.butyl | 5-(Z-amino)-pentyl |
| 27 | c-hexyl-OCH₂CH₂OCH₂ | isobutyl | tert.butyl | 6-imino-7-oxo-10-methyl-undecyl |
| 28 | c-hexyl-OCH₂CH₂OCH₂ | isobutyl | tert.butyl | morpholinocarbonylmethyl |
| 29² | c-hexyl-OCH₂CH₂OCH₂ | isobutyl | tert.butyl | 2-pyridyl |
| 30 | c-hexyl-OCH₂CH₂OCH₂ | isobutyl | p-methoxybenzyl | methyl |
| 31 | c-hexyl-OCH₂CH₂OCH₂ | isobutyl | methyl-3-indolyl | methyl |
| 32 | c-hexyl-OCH₂CH₂OCH₂ | isobutyl | 1-R-tert.butyloxy-ethyl | methyl |
| 33 | HO-CH₂CH₂OCH₂ | isobutyl | tert. butyl | methyl |
| 34 | HO-CH(benzyl)-CH₂OCH₂ | isobutyl | tert.butyl | methyl |
| 35 | HO-CH₂ | isobutyl | benzyl | methyl |
| 36 | HO-CH₂ | isobutyl | tert.butyl | methyl |
| 37 | HO-CH₂ | isobutyl | tert.butyl | 6-imino-7-oxo-10-methyl-undecyl |
| 38 | HO-CH₂ | isobutyl | tert.butyl | 6-imino-8-phenyl-octyl |
| 39 | HO-CH₂ | isobutyl | tert.butyl | 5-(morpholino carbonyl)pentyl |
| 40 | HO-CH₂ | isobutyl | tert.butyl | morpholinocarbonylmethyl |
| 41 | HO-CH₂ | isobutyl | tert.butyl | 2-(morpholino carbonyl)ethyl |
| 42 | HO-CH₂ | n-propyl | tert.butyl | methyl |
| 43 | HOCH₂ | isopropyl | tert.butyl | methyl |
| 44 | HOCH₂ | cyclopropyl | tert.butyl | methyl |
| 45 | HOCH₂ | 3-methylbutyl | tert.butyl | methyl |
| 46 | HOCH₂ | cyclopentyl | tert.butyl | methyl |
| 47 | HOCH₂ | cyclohexyl | tert-butyl | methyl |
| 48 | HOCH₂ | cyclopentyl-methyl | tert.butyl | methyl |
| 49 | HOCH₂ | cyclohexyl-methyl | tert.butyl | methyl |
| 50 | HOCH₂ | 2-methoxyethyl | tert.butyl | methyl |
| 51 | HOCH₂ | phenyl | tert.butyl | methyl |
| 52 | HOCH₂ | benzyl | tert.butyl | methyl |
| 53 | HOCH₂ | 4-phenyl-phenyl | tert.butyl | methyl |
| 54 | HOCH₂ | 2-phenylethyl | tert.butyl | methyl |
| 55 | HOCH₂ | 2-naphthyl | tert.butyl | methyl |
| 56 | HOCH₂ | 3-methyl-phenyl | tert.butyl | methyl |
| 57 | HOCH₂ | 4-methylphenyl | tert.butyl | methyl |
| 58 | HOCH₂ | 4-methoxyphenyl | tert.butyl | methyl |
| 59 | HOCH₂ | 4-fluorophenyl | tert. butyl | methyl |
| Notes to table I: Z = benzyloxycarbonyl c-hexyl = cyclohexyl 1 = unless otherwise noted, all structures have the stereochemistry of formula Ia 2 = 1/1 mixure of diasteromers related to formula Ia and Ib | | | | |

All compounds are characterized by mass-spectroscopy and proton NMR spectroscopy. Table II summerizes analytical data of example 1 - 59

**TABLE II**

| Ex | ms: [M-H]* | r.t. (A/B) | m.p. | [α]_{D}²⁰ |
|---|---|---|---|---|
| 1 | 482 | | 195-197 | -8.5 |
| 2 | 581.4 | 4.00 (10/90)) | | |
| 3 | 707.1 | | 171-172 | -3.2 |
| 4 | 609.1 | 2.98 (15/85) | 159-161 | -1.7 |
| 5 | 665.2 | 3.35 (30/70) | 211-220 | +3.6 |
| 6 | 553.4 | 1.83 (20/80) | 197-199 | -10 |
| 7 | 448.3 | | 173-174 | -11.7 |
| 8 | 561.3 | 2.33 (15/85) | | |
| 9 | 575.3 | 2.33 (15/85) | 176-177 | -8.7 |
| 10 | 617.4 | 4.44 (15/85) | 158-160 | -4.6 |
| 11 | 511.2 | 2.87 (25/75) | 197-200 | +2.8 |
| 12 | 512.1 | | 171-172 | +1.9 |
| 13 | 511.2 | | 170-171 | +24.2 |
| 14 | 496.2 | | 141-145 | +2.8 |
| 15 | 588.2 | 3.63 (30/70) | 197-199 | -5.6 |
| 16 | 521.1 | 3.26 (20/80) | 174-176 | -15.8 |
| 17 | 436.2 | 1.34 (25/75) | 115-125 | +6.8 |
| 18 | 506 | | 207 | -7.5 |
| 19 | 569 | 10.69 (30/70) | 163-166 | |
| 20 | 750.4 | 2.07 (30/70) | | |
| 21 | 647.4 | 2.51(30/70) | 167-170 | -3.3 |
| 22 | 633.3 | 2.27 (30/70) | | |
| 23 | 707.3 | 2.62 (30/70) | 161.163 | -3.5 |
| 24 | 472.2 | | 187-188 | -9.0 |
| 25 | 647.3 | 3.12 (30/70) | | |
| 26 | 677.2 | 6.54 (40/60) | 152-155 | -7.6 |
| 27 | 641.3 | 5.77 (35/65) | | |
| 28 | 585.1 | | | |
| 29 | 535.2 | 3.48 + 3.68 (40/60) | 185 | -3.5 |
| 30 | 536.2 | 3.38(30/70) | 195-200 | -8.9 |
| 31 | 545.2 | 3.56 (30/70) | 160 | -16.5 |
| 32 | 516.2 | 5.61 (30/70) | 150-170 | +26.4 |
| 33 | 390.2 | 1.73 /20/80) | 185-186 | -2.8 |
| 34 | 478.2 [M-H]⁻ | 5.78 (25/75) | 120 | -13.1 |
| 35 | 380.2 | | 173-175 | -2.5 |
| 36 | 346.1 | 2.31(10/90) | 152-155 | +2.3 |
| 37 | 515.5 | 1.92 (30/70) | 190-192 | +2.0 |
| 38 | 521.5 | 1.44 (30/70) | 173-177 | |
| 39 | 514.4 | 1.68 (20/80) | | +45.8 |
| 40 | 459 | 2.93 (20/80) | 204-206 | -23 |
| 41 | 473.3 | 1.62 (15/85) | | -3.4 |
| 42 | 332.0 | - | 144-146 | |
| 43 | 332.2 | -- | 149 - 154 | |
| 44 | | 1.18 (10/90) | 137 - 141 | +12.8 |
| 45 | | | 142-145 | -11.8 |
| 46 | 358.1 | 1.65 (20/80) | 183 - 187 | -9.4 |
| 47 | 372.2 | 1.54 (20/80) | 128 -130 | +2.0 |
| 48 | 372.1 | -- | 149 - 153 | +7.1 |
| 49 | 386.1 | 4.02 (20/80) | 142 - 144 | +4.9 |
| 50 | 348.1 | 2.56 (10/90) | -- | |
| 51 | 366.0 | 2.70 (10/90) | 152 - 155 | +20.9 |
| 52 | 380.1 | 1.92 (20/80) | 130 - 137 | +26.0 |
| 53 | 440.1 [M-H]⁻ | 2.99 (30/70) | 182-184 | +38.7 |
| 54 | 392 [M-H]⁻ | 2.73 (20/80) | 126-130 | +3.2 |
| 55 | 414.0 [M-H]⁻ | 3.14 (20/80) | 170-172 | -1.7 |
| 56 | 380.0 | 2.24 (20/80) | 152 - 153 | |
| 57 | 378.1 [M-H]⁻ | 2.13 (20/80) | 146-147 | +41.4 |
| 58 | 394.2 [M-H]⁻ | | 128-135 | +47 |
| 59 | 382.1 [M-H]⁻ | | 149-153 | +40.0 |
| Notes to table II: ms = mass spectroscopy [M-H]* = [M-H]⁺ unless otherwise indicated r.t. retetion time in min., HPLC, C18 column A = % solvent A: acetonitrile (100 %) B = % solvent B: water(88 %) + acetonitrile (9.8%) + 10% aqu. Me₄NOH (2%) + 85% aqu. H₃PO₄ (0.2%) m.p. = melting point in °C [α]_{D}²⁰ = specific optical rotation at 20°C in methanol | | | | |

### Test Example 1: Inhibition of TNF release

Mononuclear cells are prepared from the peripheral blood of healthy volunteers using ficoll-hypaque density separation according to the method of Hansell et al., J. Imm. Methods (1991) **145:** 105. and used at a concentration of 10⁵ cells/well in RPMI 1640 plus 10% PCS. Cells are incubated with serial dilutions of the test compounds for 30 minutes at 37°C prior to the addition of IFNγ (100 U/ml) and LPS (5 µg/ ml) and subsequently further incubated for three hours. Incubation is terminated by centrifugation at 1400 RPM for 10 min. TNFα in the supernatant is measured using a commercial
ELISA (Innotest hTNFα, available from Innogenetics N.V., Zwijnaarde, Belgium). Novel Compounds are tested at concentrations of from 0 to 10 µM. Exemplified compounds of formula I, especially of formula Ia, suppress TNF release in this assay with an IC₅₀ of from about 50 nM to about 5 µM.

### Test Example 2: Cytotoxicity

Cytotoxicity is determined on THP1 cells (5 x 10⁴ / well) which are incubated in the presence of IFNγ (100 U/ml) and LPS (5 µg/ ml) and presence and absence of test compound for 24 hours at 37°C. Percentages of living and dead cells are assessed by a colorimetric readout (MTT), which measures mitochondrial dehydrogenase enzymes in living cells, as described in Mosman, J. Imm. Methods (1983) **65**: 55. Novel Compounds tested show less than 50% cytotoxicity at a concentration of 10 µM, showing that the Novel Compounds are not cytotoxic at concentrations sufficient to suppress TNF.

### Test Example 3: Collagenase inhibition

Collagenase inhibition is determined using active collagenase with the thiopeptide MMP-substrate described in Stein and Izquierdo-Martin, Arch. Biochem. Biophys. 308 (1994) pp. 274-277. Test compound is incubated with the collagenase prior to the addition of the substrate at pH 6.5, 25°C in 2-morpholinoethanesulphonic acid (50mM) buffer with 10mM CaCl₂. The absorbance is recorded at 405nm at regular intervals for a period of 40 minutes. The inhibitory activity of the test compound is determined as a function of the collagenase activity in the control in the presence and absence of the test compound. The Novel Compounds show significant dose dependent inhibition of collagenase at low nM concentrations, e.g., below 10 nM.

### Test Example 4: Oral bioavailability

The assay of the preceding example is standardized by measuring activity of varying known concentrations of a particular test compound and used to measure the concentration of test compound in plasma following oral administration. Test compounds are admistered orally to conscious rats at a dosage of 10 mg/kg. Blood samples are taken from the cut tip of the tail at 30, 60, 120, and 240 minutes from oral administration. The plasma is subjected to trichloroacetic acid extraction. The extract is tested in the above collagenase inhibition assay to obtain an estimate of the concentration of drug present in the plasma. The Novel Compounds show good oral bioavailability, with plasma concentrations of 300-5000 nM after 30 minutes and 50-500 nM after 240 minutes. Thus, pharmaceutically effective plama levels (as shown in Test Example 1 and 3) are readily achievable with oral administration at manageable dosages, e.g., 10 mg/kg. Moreover, the plasma levels obtained are well below the cytotoxic level, and the rats were not observed to show any adverse effects at this dosage.

## Claims

1. A 3-imino-4-oxo-6-(oxymethyl)-heptane-1,7-dioic acid (7-N-hydroxy) diamide, in free or pharmaceutically acceptable salt form.

2. A compound according to claim 1 which is a 3-imino-4-oxo-5-aryl-6-(oxymethyl)-heptane-1,7-dioic acid (7-N-hydroxy) diamide, in free or pharmaceutically acceptable salt form.

3. A compound according to claim 1 of Formula I 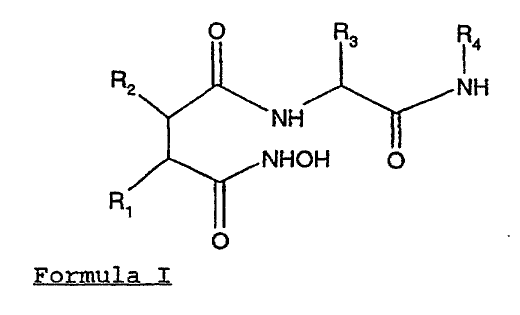 wherein
**R**_{**1**} is a substituent of Formula II:
A-(O-(CR₅H)ₙ)ₘ-O-CH₂- *Formula II*
wherein
**n** is 1, 2, 3 or 4;
**m** is 0, 1, 2 or 3;
each **R**_{**5**} is
independently H, C₁₋₁₀ (optionally hydroxy-, C₁₋₆ alkoxy-, amino-, C₁₋₆ alkylamino-, thiol-, C₁₋₆ alkylmercapto- or protected hydroxy, amino or thiol substituted) alkyl, C₂₋₆ alkenyl, C₆₋₁₄(optionally hydroxy-, C₁₋₆ alkoxy-, amino-, C₁₋₆ alkylamino-, halo- or cyano- substituted) aryl, or C₆₋₁₄ (aryl) C₁₋₆alkyl;
**A** is hydrogen, C₁₋₁₀ alkyl, C₆₋₁₄ aryl, C₆₋₁₄ aryl(C₁₋₆ alkyl), (C₆₋₁₄ aryl)carbonyl, or (C₁₋₁₀ alkyl)carbonyl;
**R**_{**2**} is C₃₋₁₂ alkyl, C₃₋₁₂ alkenyl, C₃₋₇(optionally hydroxy-, C₁₋₆ alkoxy-, amino-, or C₁₋₆ alkylamino- substituted) cycloalkyl, C₅₋₁₄ aryl, or C₅₋₁₄ aryl(C₁₋₆ alkyl), wherein aryl groups are optionally substituted by hydroxy-, C₁₋₆ alkyl-, C₁₋₆ alkoxy-, amino-, halo- or cyano-;
**R**_{**3**} is C₁₋₁₀ (optionally hydroxy- or C₁₋₆alkoxy- amino-, C₁₋₆ alkylamino-, thiol-, C₁₋₆ alkylmercapto- or protected hydroxy-, amino- or thiol- substituted) alkyl, C₆₋₁₄ (optionally hydroxy-, C₆₋₁₄aryloxy-, or C₁₋₆alkoxy-, amino-, C₁₋₆ alkylamino-, halo-, or cyano- substituted)aryl, or indolylmethyl;
**R**_{**4**} is methyl, pyridyl, or a substituent of formula X-Y- wherein X is morpholino, pyridyl or aryl, and Y is C₁₋₁₂alkylene in which up to four of the methylene (-CH₂-) units are optionally replaced with -CO-, -NH-, -SO₂- or -O-,
in free or pharmaceutically acceptable salt form.

4. A compound according to claim 3 in which R₁ is a substituent of Formula II'
A-(O-(CH₂)ₙ)ₘ-O-CH₂- *Formula II'*
wherein A, n and m are as defined in claim 3.

5. A compound according to claim 3 in which R₁ is a substituent of Formula II"
A-O-CHR₅-(O-(CH₂)ₙ)ₘ,-O-CH₂- *Formula II"*
wherein A, n and R₅ are as defined in claim 3 and m' is 0, 1 or 2.

6. A compound according to claim 3, 4 or 5 with the proviso that when m of formula II is zero, then R₄ of formula I is a substituent of formula X-Y-

7. A compound of formula I as defined in claim 3 in which independently:
n of Formula II is 3 or 4; or
**R**_{**5**} of Formula II is not H; or
**R**_{**2**} is C₇₋₁₂ alkyl, C₃₋₁₂ alkenyl, C₃₋₇ (optionally hydroxy-, C₁₋₆ alkoxy-, amino-, or C₁₋₆ alkylamino- substituted) cycloalkyl, C₅₋₁₄ aryl, or C₅₋₁₄ aryl(C₁₋₆ alkyl), wherein aryl groups are optionally substituted by hydroxy-, C₁₋₆ alkyl-, C₁₋₆ alkoxy-, amino-, halo- or cyano-; or
**R**_{**3**} is C₁₋₁₀(amino-, C₁₋₆ alkylamino-, thiol-, C₁₋₆ alkylmercapto- or protected hydroxy-, amino- or thiol- substituted)alkyl, C₆₋₁₄(amino-, C₁₋₆ alkylamino-, halo-, or cyano-substituted)aryl; or
any aryl group thereof is heteroaryl containing one or more hetero atoms, e.g. N, O or S.

8. A compound according to claim 3 of formula I' 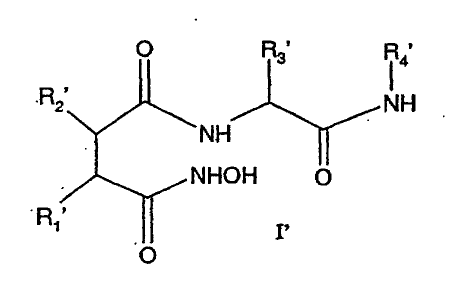 in which
**R**_{**1**}**'** is a substituent of Formula II"':
A'-(O-(CH₂)_{n'})_{m'}-O-CH₂- *Formula II'''*
such that
**n'** is an integer one or two, preferably two;
**m'** is an integer zero, one, two, or three;
**A'** is hydrogen, C₆₋₁₄ aryl, C₁₋₁₀ alkyl, (C₆₋₁₄ aryl)carbonyl, or (C₁₋₁₀ alkyl)carbonyl;
**R**_{**2**}**'** is C₂₋₆ alkyl;.
**R**_{**3**}**'** is C₁₋₁₀ (optionally hydroxy- or C₁₋₆alkoxy-substituted) alkyl, C₆₋₁₄ (optionally hydroxy-, C₆₋₁₄aryloxy-, or C₁₋₆alkoxy-substituted) aryl, or indolylmethyl;
**R**_{**4**}**'** is methyl, pyridyl, or a substituent of formula X-Y- wherein X is morpholino, pyridyl or aryl, and Y is C₁₋₁₂alkylene in which up to four of the methylene (-CH₂-) units are optionally replaced with -CO-, -NH-, -SO₂- or -O-,
in free or pharmaceutically acceptable salt form.

9. A compound according to any one of claims 3 to 7 wherein:
(i) **R**_{**1**} is of formula II' or II" and A of formula II is hydrogen, C₁₋₆ alkyl, or (C₆₋₁₄ aryl)carbonyl;
(ii) R₂ of formula I is cyclohexyl, phenyl, 4-methylphenyl, 4-methoxyphenyl or isobutyl;
(iii) R₃ of formula I is benzyl or *t*-butyl; and
(iv) R₄ of formula I is methyl or morpholinocarbonyl(C₁₋₆)alkyl.

10. A compound according to any of claims 1 - 9 where the configuration is of Formula Ia:

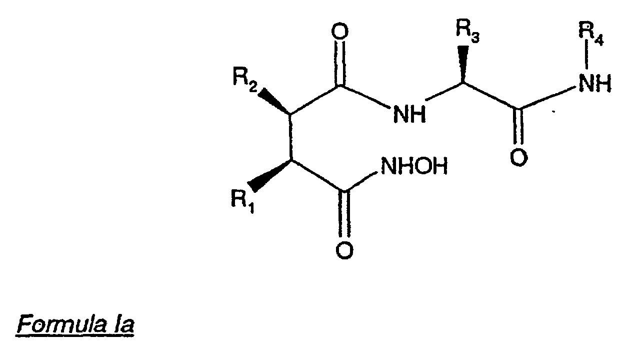

11. A compound according to any one of claims 1-10 for use as a pharmaceutical.

12. A pharmaceutical composition comprising a compound according to any one of claims 1-10.

13. Use of a compound according to any one of claims 1-10 in the manufacture of a medicament for the treatment of an autoimmune or inflammatory disease or condition.

14. A compound of Formula III 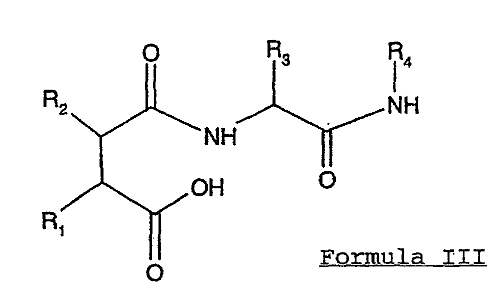 wherein R₁, R₂, R₃ and R₄ are as defined in claim 3.

15. A compound of formula IV 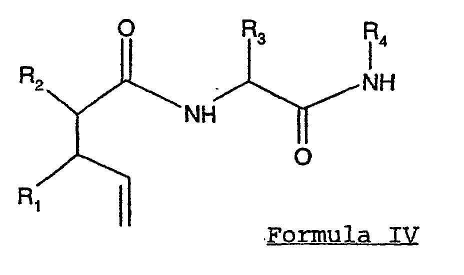 wherein R₁, R₂, R₃ and R₄ are as defined in claim 3.

16. A process for making a compound according to any of claims 1-11 comprising the steps of reacting a compound of Formula III according to claim 16 with hydroxylamine (optionally in salt or O-protected form) and, if required, deprotecting the product thus obtained.

## Patentansprüche

1. 3-Imino-4-oxo-6-(oxymethyl)-heptan-1,7-dicarbonsäure-(7-N-hydroxy)diamid in freier Form oder in pharmazeutisch annehmbarer Salzform.

2. Verbindung nach Anspruch 1, die 3-Imino-4-oxo-5-aryl-6-(oxymethyl)-heptan-1,7-dicarbonsäure-(7-N-hydroxy)diamid in freier Form oder in pharmazeutisch annehmbarer Salzform ist.

3. Verbindung nach Anspruch 1 der Formel I 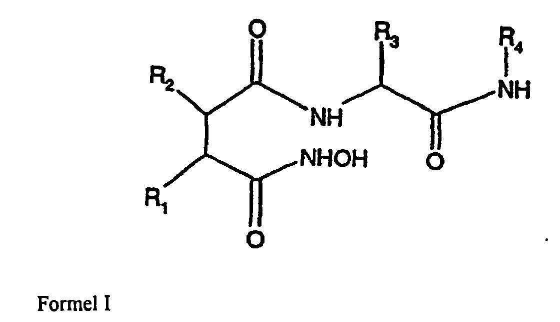 worin
R₁ für einen Substituenten der Formel II steht
A-(O-(CR₅H)ₙ)ₘ-O-CH₂- Formel II
worin
n für 1, 2, 3 oder 4 steht.
m für 0, 1, 2 oder 3 steht,
jedes R₅ unabhängig steht für H, C₁-C₁₀ (wahlweise substituiert mit Hydroxy, C₁-C₆ Alkoxy, Amino, C₁-C₆ Alkylamino, Thiol, C₁-C₆ Alkylmercapto oder geschützem Hydroxy, Amino oder Tluol) Alkyl, C₂-C₆ Alkenyl. C₆-C₁₄ (wahlweise substituiert mit Hydroxy, C₁-C₆ Alkoxy, Amino, C₁-C₆ Alkylamino, Halogen oder Cyano) Aryl oder C₆-C₁₄ (Aryl)-C₁-C₆-alkyl,
A für Wasserstoff, C₁-C₁₀ Alkyl, C₆-C₁₄ Aryl, C₆-C₁₄ Aryl(C₁-C₆-alkyl), (C₆-C₁₄ Aryl)carbonyl oder (C₁-C₁₀ Alkyl)carbonyl steht,
R₂ für C₃-C₁₂ Alkyl, C₃-C₁₂ Alkenyl. C₃-C₇ (wahlweise substituiert mit Hydroxy, C₁-C₆ Alkoxy, Amino oder C₁-C₆ Alkylamino) Cycloalkyl, C₅-C₁₄ Aryl oder C₅-C₁₄ Aryl(C₁-C₆-alkyl) steht, worin die Arylgruppen wahlweise mit Hydroxy, C₁-C₆ Alkyl, C₁-C₆ Alkoxy, Amino, Halogen oder Cyano substituiert sind,
R₃ steht für C₁-C₁₀ (wahlweise substituiert mit Hydroxy, C₁-C₆ Alkoxy, Amino, C₁-C₆ Alkylamino, Thiol, C₁-C₆ Alkylmercapto oder geschützem Hydroxy, Amino oder Thiol) Alkyl, C₆-C₁₄ (wahlweise substituiert mit Hydroxy, C₆-C₁₄ Aryloxy oder C₁-C₆ Alkoxy, Amino, C₁-C₆ Alkylamino, Halogen oder Cyano) Aryl oder Indolylmethyl.
R₄ für Methyl, Pyridyl oder einen Substituenten der Formel X-Y- steht, worin X für Morpholino, Pyridyl oder Aryl steht und Y für C₁-C₁₂ Alkylen steht, worin bis zu vier der Methyleneinheiten (-CH₂-) wahlweise ersetzt sind durch -CO-, -NH-, -SO₂- oder -O-,
in freier Form oder pharmazeutisch annehmbarer Salzform.

4. Verbindung nach Anspruch 3, worin R₁ für einen Substituenten der Formel II' steht
A-(O-(CH₂)ₙ)ₘ-O-CH₂- Formel II'
worin A, n und m wie in Anspruch 3 definiert sind.

5. Verbindung nach Anspruch 3, worin R₁ für einen Substituenten der Formel II" steht
A-O-CHR₅-(O(CH₂)ₙ)_{m'}-O-CH₂- Formel IIʺ
worin A, n und R₅ wie in Anspruch 3 definiert sind und m' für 0, 1 oder 2 steht.

6. Verbindung nach Anspruch 3, 4 oder 5, mit der Maßgabe, daß wenn m der Formel II für 0 steht, R₄ der Formel I dann für einen Substituenten der Formel X-Y- steht.

7. Verbindung der Formel I nach Anspruch 3, worin unabhängig
n der Formel II für 3 oder 4 steht, oder
R₅ der Formel II nicht für H steht, oder
R₂ für C₇-C₁₂ Alkyl, C₃-C₁₂ Alkenyl, C₃-C₇ (wahlweise substituiert mit Hydroxy, C₁-C₆ Alkoxy, Amino oder C₁-C₆ Alkylamino) Cycloalkyl, C₅-C₁₄ Aryl oder C₅-C₁₄ Aryl(C₁-C₆-alkyl) steht, worin die Arylgruppen wahlweise mit Hydroxy, C₁-C₆ Alkyl, C₁-C₆ Alkoxy, Amino, Halogen oder Cyano substituiert sind, oder
R₃ steht für C₁-C₁₀ (substituiert mit Amino, C₁-C₆ Alkylamino, Thiol, C₁-C₆ Alkylmercapto oder geschützem Hydroxy, Amino oder Thiol) Alkyl, C₆-C₁₄ (substituiert mit Amino, C₁-C₆ Alkylamino, Halogen oder Cyano) Aryl, oder eine Arylgruppe hiervon ein Heteroaryl ist, das ein oder mehrere Heteroatome enthält, beispielsweise N, O oder S.

8. Verbindung nach Anspruch 3 der Formel I' 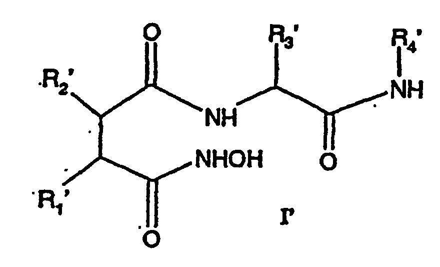 worin
R₁' für einen Substituenten der Formel II''' steht,
A'-(O-(CH₂)_{n'})_{m'}-O-CH₂- Formel II'''
so daß
n' für eine ganze Zahl von 1 oder 2, vorzugsweise 2 steht,
m' für eine ganze Zahl von 1, 2 oder 3 steht,
A' für Wasserstoff, C₆-C₁₄ Aryl, C₁-C₁₀ Alkyl, (C₆-C₁₄ Aryl)carbonyl oder (C₁-C₁₀ Alkyl)carbonyl steht,
R₂' für C₂-C₆ Alkyl steht,
R₃' für C₁-C₁₀ (wahlweise substituiert mit Hydroxy oder C₁-C₆ Alkoxy) Alkyl, C₆-C₁₄ (wahlweise substituiert mit Hydroxy, C₆-C₁₄ Aryloxy oder C₁-C₆ Alkoxy) Aryl oder Indolylmethyl steht,
R₄' für Methyl, Pyridyl oder einen Substituenten der Formel X-Y- steht, worin X für Morpholino, Pyridyl oder Aryl steht und Y für C₁-C₁₂ Alkylen steht, worin bis zu vier der Methyleneinheiten (-CH₂-) wahlweise ersetzt sind durch -CO-, -NH-, -SO₂- oder -O-,
in freier Form oder pharmazeutisch annehmbarer Salzform.

9. Verbindung nach einem der Ansprüche 3 bis 7, worin
(i) R₁ die Formel II' oder II" hat und A der Formel II für Wasserstoff, C₁-C₆ Alkyl oder (C₆-C₁₄ Aryl)carbonyl steht,
(ii) R₂ der Formel I für Cyclohexyl, Phenyl, 4-Methylphenyl, 4-Methoxyphenyl oder Isobutyl steht,
(iii) R₃ der Formel I für Benzyl oder t-Butyl steht, und
(iv) R₄ der Formel I für Methyl oder Morpholinocarbonyl(C₁-C₆)alkyl steht.

10. Verbindung nach einem der Ansprüche 1 - 9, worin die Konfiguration die der Formel Ia ist

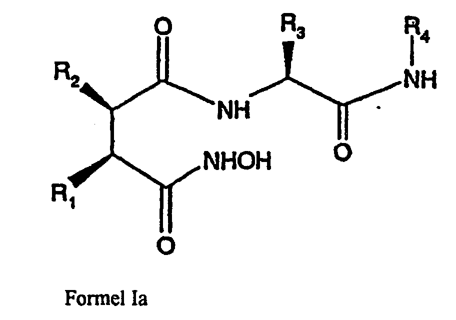

11. Verbindung nach einem der Ansprüche 1 - 10 zur Verwendung als Pharmazeutikum.

12. Pharmazeutische Zusammensetzung, die eine Verbindung nach einem der Ansprüche 1 - 10 enthält.

13. Verwendung einer Verbindung nach einem der Ansprüche 1 - 10 zur Herstellung eines Arzneimittels zur Behandlung einer autoimmunen oder entzündlichen Erkrankung oder eines solchen Zustands.

14. Verbindung der Formel III 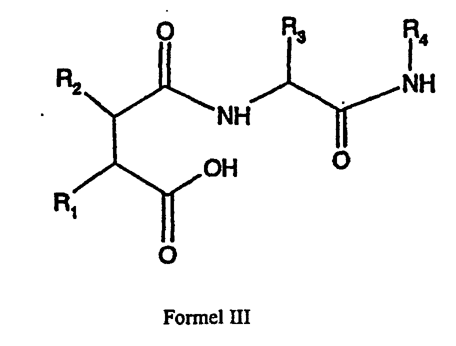 worin R₁, R₂, R₃ und R₄ wie in Anspruch 3 definiert sind.

15. Verbindung der Formel IV 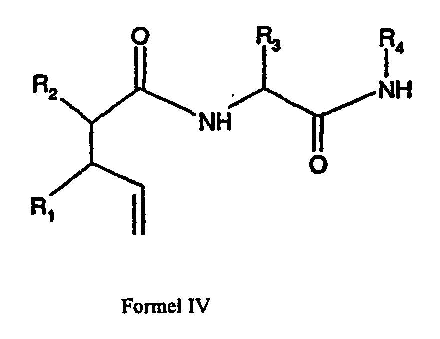 worin R₁, R₂, R₃ und R₄ wie in Anspruch 3 definiert sind.

16. Verfahren zur Herstellung einer Verbindung nach einem der Ansprüche 1-11, das die Schritte der Umsetzung einer Verbindung der Formel III nach Anspruch 16 mit Hydroxylamin (wahlweise in Salzform oder O-geschützter Form) und erforderlichenfalls der Schutzgruppenabspaltung des so erhaltenen Produkts umfaßt.

## Revendications

1. (7-N-hydroxy)diamide d'acide 3-imino-4-oxo-6-(oxyméthyl)-heptane-1,7-dioïque, sous forme libre ou sous forme d'un sel pharmaceutiquement acceptable.

2. Composé suivant la revendication 1, qui est un (7-N-hydroxy)diamide d'acide 3-imino-4-oxo-5-aryl-6-(oxyméthyl)-hcptane-1,7-dioïque, sous forme libre ou sous forme d'un sel pharmaceutiquement acceptable.

3. Composé suivant la revendication 1, de formule I: 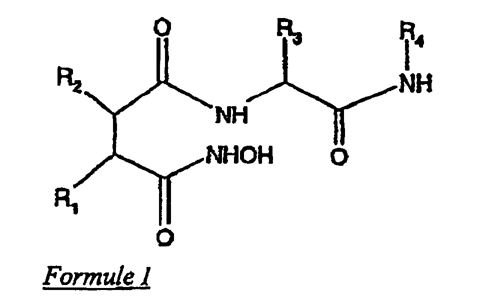 dans laquelle
R₁ est un substituant de formule II :
A-(O-(CR₅H)ₙ)ₘ-O-CH₂ *Formule II*
où
n vaut 1, 2, 3 ou 4 ;
m vaut 0, 1, 2 ou 3 ;
chaque radical R₅ représente indépendamment H, un alkyle en C₁ à C₁₀ (éventuellement à substitution hydroxy-, alcoxy en C₁ à C₆-, amino-, alkyle en C₁ à C₆-amino-, thiol-, alkyle en C₁ à C₆-mercapto- ou hydroxy-protégé, amino- ou thiol-), alcényle en C₂ à C₆, aryle en C₆ à C₁₄ (éventuellement à substitution hydroxy-, alcoxy en C₁ à C₆, amino-, alkyle en C₁ à C₆-amino-, halo- ou cyano-) ou aryle en C₆ à C₁₄-alkyle en C₁ à C₆ ;
A représente l'hydrogène, un alkyle en C₁ à C₁₀, aryle en C₆ à C₁₄, aryle en C₆ à C₁₄-(alkyle en C₁ à C₆), (aryle en C₆ à C₁₄)carbonyle ou (alkyle en C₁ à C₁₀)-carbonyle ;
R₂ représente un alkyle en C₃ à C₁₂, alcényle en C₃ à C₁₂, cycloalkyle en C₃ à C₇ (éventuellement à substitution hydroxy-, alcoxy en C₁ à C₆-, amino- ou alkyle en C₁ à C₆-amino-), aryle en C₅ à C₁₄, ou aryle en C₅ à C₁₄-(alkyle en C₁ à C₆), dans lequel les groupes aryle sont éventuellement substitués par un hydroxy, alkyle en C₁ à C₆, alcoxy en C₁ à C₆, amino, halo, ou cyano ;
R₃ représente un alkyle en C₁ à C₁₀ (éventuellement à substitution hydroxy- ou alcoxy en C₁ à C₆-amino-, alkyle en C₁ à C₆-amino-, thiol-alkyle en C₁ à C₆-mercapto-, ou hydroxy-protégé, amino- ou thiol-), aryle en C₆ à C₁₄ (éventuellement à substitution hydroxy-, aryloxy en C₆ à C₁₄-, ou alcoxy en C₁ à C₆-, amino-, alkyle en C₁ à C₆-amino-, halo- ou cyano-), ou indolylméthyle ;
R₄ représente un méthyle, pyridyle, ou un substituant de formule X-Y- dans laquelle X est un morpholino, pyridyle ou aryle, et Y est un alkylène en C₁ à C₁₂ dans lequel jusqu'à quatre des motifs méthylène (-CH₂-) sont éventuellement remplacés par -CO-, -NH-, -SO₂- ou -O-, sous forme libre ou sous forme d'un sel pharmaceutiquement acceptable.

4. Composé suivant la revendication 3, dans lequel R₁ est un substituant de formule II' :
A-(O-(CH₂)ₙ)ₘ-O-CH₂- *Formule II'*
dans laquelle A, n et m sont tels que définis dans la revendication 3.

5. Composé suivant la revendication 3, dans lequel R₁ est un substituant de formule II" :
A-O-(CHR₅-(CH₂)ₙ)_{m'}-O-CH₂- *Formule II"*
dans laquelle A, n et R₅ sont tels que définis dans la revendication 3, et m' vaut 0, 1 ou 2.

6. Composé suivant la revendication 3, 4 ou 5, sous réserve que lorsque m dans la formule II vaut zéro, R₄ dans la formule 1 est un substituant de formule X-Y-,.

7. Composé de formule I tel que défini dans la revendication 3, dans lequel, de manière indépendante :
n dans la formule II vaut 3 ou 4 ; ou
R₅ dans la formule II ne représente pas H ; ou
R₂ est un alkyle en C₇ à C₁₂, alcényle en C₃ à C₁₂, cycloalkyle en C₃ à C₇ (éventuellement à substitution hydroxy-, alcoxy en C₁ à C₆-, amino-, ou alkyle en C₁ à C₆-amino-), aryle en C₅ à C₁₄, ou aryle en C₅ à C₁₄-(alkyle en C₁ à C₆), où les groupes aryle sont éventuellement substitués par un hydroxy, alkyle en C₁ à C₆, alcoxy en C₁ à C₆, amino, halo ou cyano ; ou
R₃ est un alkyle en C₁ à C₁₀ (à substitution amino-, alkyle en C₁ à C₆-amino-, thiol-, alkyle en C₁ à C₆-mercapto-, ou hydroxy-protégé, amino- ou thiol-), aryle en C₆ à C₁₄ (à substitution amino-, alkyle en C₁ à C₆-amino, halo- ou cyano-) ; ou bien
n'importe quel groupe aryle est un hétéroaryle contenant un ou plusieurs hétéroatomes, par exemple N, O ou S.

8. Composé suivant la revendication 3 de formule I' : 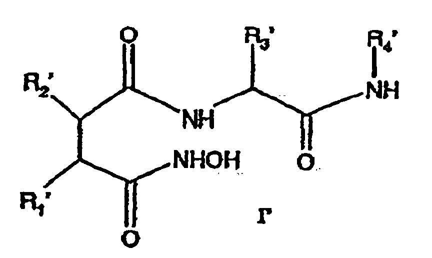 dans laquelle :
R₁' est un substituant dc formule II''' :
A'-(O-(CH₂)_{n'})_{m'}-O-CH₂- *Formule II'''*
où
n' est le nombre entier 1 ou 2, de préférence 2 ;
m' est le nombre entier 0, 1, 2 ou 3 ;
A' représente l'hydrogène, un aryle en C₆ à C₁₄, alkyle en C₁ à C₁₀, (aryle en C₆ à C₁₄)-carbonyle ou (alkyle en C₁ à C₁₀)carbonyle ;
R₂' représente un alkyle en C₂ à C₆ ;
R₃' représente un alkyle en C₁ à C₁₀ (éventuellement hydroxy- ou alcoxy en C₁ à C₆-substitué), un aryle en C₆ à C₁₄ (éventuellement hydroxy-, aryloxy en C₆ à C₁₄- ou alcoxy en C₁ à C₆-substitué), ou indolylméthyle ;
R₄' représente un méthyle, pyridyle, ou un substituant de formule X-Y- dans laquelle X est un morpholino, pyridyle ou aryle, et Y est un alkylène en C₁ à C₁₂ dans lequel jusqu'à quatre des motifs méthylène (-CH₂-) sont évenluellement remplacés par -CO-, -NH-, -SO₂-, ou -O- ; sous forme libre ou sous forme d'un sel pharmaceutiquement acceptable.

9. Composé suivant l'une quelconque des revendications 3 à 7, dans lequel :
(i) R₁ répond à la formule II' ou II" et Λ dans la formule II représente l'hydrogène, un alkyle en C₁ à C₆, ou (aryle en C₆ à C₁₄)-carbonyle ;
(ii) R₂ dans la formule I est un cyclohexyle, phényle, 4-méthylphényle, 4-méthoxyphénylc ou isobutyle ;
(iii) R₃ dans la formule I est un benzyle ou tert-butyle ; et
(iv) R₄ dans la formule I est un méthyle ou morpholino-carbonyl-(alkyle en C₁ à C₆).

10. Composé suivant l'une quelconque des revendications 1 à 9, dans lequel la configuration est celle de formule Ia ;

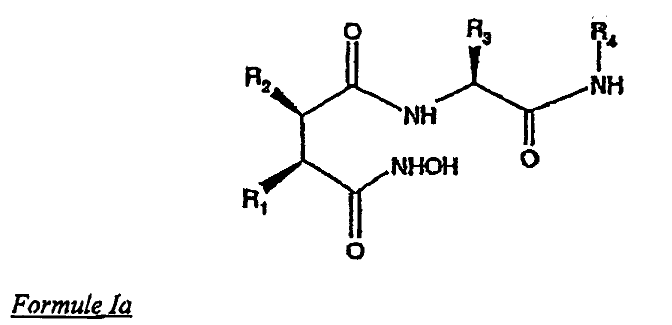

11. Composé suivant l'une quelconque des revendications 1 à 10, pour utilisation comme produit pharmaceutique.

12. Composition pharmaceutique comprenant un composé suivant l'une quelconque des revendications 1 à 10.

13. Utilisation d'un composé suivant l'une quelconque des revendications 1 à 10, dans la fabrication d'un médicament pour le traitement d'une maladie ou d'une affection auto-immune ou inflammatoire.

14. Composé de formule III: 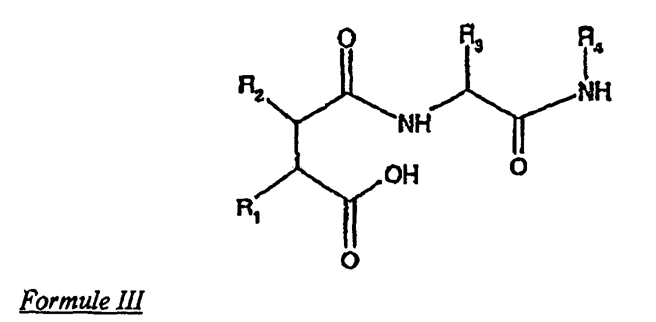 dans laquelle R₁, R₂, R₃ et R₄ sont tels que définis dans la revendication 3.

15. Composé de formule IV : 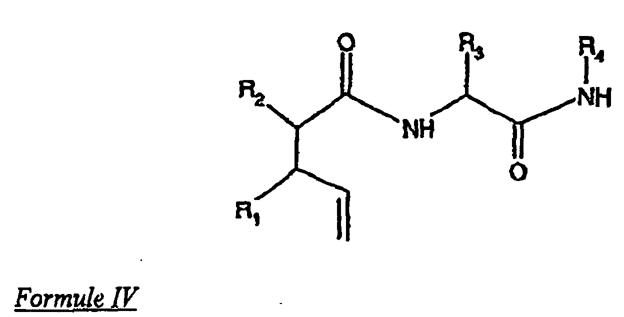 dans laquelle R₁, R₂, R₃ et R₄ sont tels que définis dans la revendication 3.

16. Procédé de préparation d'un composé suivant l'une quelconque des revendications 1 à 11, comprenant les étapes consistant à faire réagir un composé de formule III suivant la revendication 16 avec une hydroxylamine (éventuellement sous la forme d'un sel ou sous forme O-protégée) et, si cela est nécessaire, à supprimer la protection du produit ainsi obtenu.
